# FASCICULE DE BREVET EUROPEEN

(11) **EP 2 662 127 B1**
(45) Date de publication et mention de la délivrance du brevet: **28.01.2015**
(21) Numéro de dépôt: 13305436.1
(22) Date de dépôt: 04.04.2013
(51) Int. Cl.: B01D 53/86

(54) **Procede de traitement de gaz avec hydrolyse catalytique de COS et/ou CS2**
Gasaufbereitungsverfahren mit katalytischer Hydrolyse von COS und/oder CS2
Gas treatment method with catalytic hydrolysis of COS and/or CS2

(30) Priorité: 10.05.2012 FR 1201362
(43) Date de publication de la demande: 13.11.2013
(73) Titulaire: AXENS, 92508 Rueil Malmaison Cedex (FR)
(72) Inventeur: Roisin, Eric, 92500 Rueil-Malmaison (FR); Lambert, Nicolas, 92130 Issy-les-Moulineaux (FR); Loonis, Yann, 75015 Paris (FR)
(74) Mandataire: Schmitt, Nicolas A.J.

(56) Documents cités:
- WO-A1-2006/055326
- WO-A1-2006/065459
- WO-A1-2009/020473
- GB-A- 563 350
- US-A- 3 084 023

## Description

### Domaine de l'invention

L'invention se rapporte à un procédé de traitement de gaz qui comprend notamment comme impuretés à éliminer des composés soufrés. En particulier, le procédé s'applique au traitement de gaz effluent issu des fourneaux d'unité de fabrication de noir de charbon.

### État de la technique

Il existe divers procédés industriels qui génèrent l'émission des effluents gazeux contenant du soufre. Ces effluents ou gaz résiduaires doivent être traités avant leur rejet dans l'atmosphère afin d'amener la quantité de ces composés soufrés polluants à des niveaux acceptables, de manière à satisfaire aux normes environnementales de plus en plus exigeantes.

Le soufre rencontré dans les gaz résiduaires industriels, par exemple dans les gaz issus d'unités de gazéification du charbon ou de petcoke ou encore de biomasse ou dans les gaz issus des fours de calcination des unités de fabrication de noir de carbone, se trouvent généralement sous forme d'anhydride sulfureux, d'hydrogène sulfuré et de sulfure de carbone, tels que le disulfure de carbone (CS₂) et de sulfure de carbonyle (COS). Les sulfures de carbone sont des composés relativement inertes et restent donc difficiles à éliminer de l'effluent de manière efficace.

On connaît dans l'état de la technique différentes méthodes pour éliminer le soufre. Ces méthodes sont généralement fondées sur le principe de formation d'hydrogène sulfuré (H₂S) qui est un composé réactif et aisément éliminable.

Ainsi par exemple le document US 5,466,427 divulgue un procédé de traitement d'un gaz résiduaire contenant du soufre, notamment sous forme de sulfure de carbone, qui consiste à mettre en contact ledit gaz avec un catalyseur afin de réaliser une hydrolyse du disulfure de carbone (CS₂) et du sulfure de carbonyle (COS) en hydrogène sulfuré. Le catalyseur utilisé dans ce même document comprend:
- 0,5 - 5% en poids d'oxyde de cobalt et/ou de nickel;
- 1,5 - 15% en poids de trioxyde de molybdène
- 70 - 98% en poids d'oxyde de titane.

Comme observé par la demanderesse, le procédé de l'art antérieur fonctionne correctement à condition que le gaz à traiter comporte une quantité relativement faible de produits hydrocarbures insaturés (notamment des composés du type alcyne et diène), c'est-à-dire à une concentration maximum de 50 ppm, voire 30 ppm en volume.

WO2006/065459 divulgue un procédé de traitement d'un gaz comprenant au moins l'un des composés COS et CS2 et des composés hydrocarbures insaturés.

### Description

Un but de l'invention est de proposer un procédé de traitement d'un gaz capable de transformer les composés soufrés présents notamment sous forme de CS₂ et/ou COS qui soit opérationnel même lorsque la charge à traiter contient des composés hydrocarbure insaturés, par exemple à une concentration supérieure à 30 ppm en volume.

A cette fin, le procédé selon l'invention comprend les étapes suivantes:
a) on effectue une hydrogénation des composés hydrocarbures insaturés en paraffines, en mettant en contact ledit gaz avec un catalyseur d'hydrogénation en présence d'hydrogène à une température comprise entre 100 et 400°C, dé manière à fournir un effluent appauvri en composés hydrocarbures insaturés, le catalyseur d'hydrogénation comprenant au moins un métal choisi parmi le palladium, le platine, le nickel et le cobalt déposé sur un support poreux,
b) on effectue une hydrolyse catalytique en présence d'eau du COS et/ou du CS₂ présents dans l'effluent issu de l'étape a) de manière à fournir un effluent riche en H₂S en mettant en contact l'effluent issu de l'étape a) avec un catalyseur d'hydrolyse, le catalyseur d'hydrolyse comprenant de l'alumine ou de l'oxyde de titane.

Il a été ainsi observé que l'étape a) d'hydrogénation de la charge permet de prévenir la désactivation progressive, voire un bouchage du catalyseur d'hydrolyse de l'étape b), suite notamment à la formation de gommes de polymérisation à la surface des catalyseurs. En effet grâce au prétraitement de la charge à l'étape a) on transforme les composés hydrocarbures insaturés par hydrogénation en des composés qui ne sont pas susceptibles de polymériser et donc d'engendrer un empoisonnement, un cokage ou un bouchage des pores du catalyseur d'hydrolyse lors de l'étape b) subséquente d'où une efficacité améliorée du procédé de traitement par rapport à l'art antérieur.

Dans le contexte de l'invention, le terme "composés hydrocarbures insaturés" regroupe notamment les composées du type alcène, alcyne et polyinsaturés du type diènes.

Selon un mode de réalisation, les étapes a) et b) sont effectuées dans un même réacteur dans lequel sont disposés successivement deux lits de catalyseur, à savoir un catalyseur d'hydrogénation et un catalyseur d'hydrolyse. Les lits sont agencés l'un par rapport à l'autre dans le réacteur de sorte que la charge à traiter rencontre le lit de catalyseur d'hydrogénation avant le lit de catalyseur d'hydrolyse.

Conformément à un autre mode de réalisation alternatif, le procédé met en oeuvre deux réacteurs (i.e. un réacteur d'hydrogénation et un réacteur d'hydrolyse) spécifiques et dans lequel le réacteur d'hydrogénation est installé en aval du réacteur d'hydrolyse.

Selon un mode de réalisation préféré, le procédé selon l'invention comprend une étape de traitement du gaz issu de l'étape d'hydrolyse qui consiste par exemple à piéger l'H₂S formé ou à convertir l'H₂S en soufre élémentaire.

Selon un mode de réalisation avantageux, on peut procéder à une étape de séparation liquide/gaz du gaz à traiter avant d'effectuer l'étape d'hydrogénation.

De même il est possible d'effectuer une séparation liquide/gaz du gaz issu de l'étape b) avant de l'envoyer vers une unité de traitement de l'H₂S.

### La charge gazeuse à traiter

Le gaz qui peut être traité par le procédé selon l'invention peut être issu d'unités de gazéification du charbon ou de petcoke ou encore de biomasse ou des fours de calcination des unités de fabrication de noir de carbone. Typiquement le gaz à traiter peut comprendre du COS et/ou du CS₂ à une teneur comprise entre 10 ppm en volume et 0,5% volume. Le gaz comprend ainsi généralement du COS à une teneur le plus souvent comprise entre 10 ppm en volume et 0,3% volume, du CS₂ à une teneur comprise entre 10 ppm volume et 0,3% volume et éventuellement de l'HCN à une teneur comprise entre 20 ppm en volume et 0,2% volume. Le gaz peut également comprendre de l'hydrogène, du CO, du SO₂, du CO₂, de l'H₂S et de l'eau.

Le gaz comprend généralement des hydrocarbures insaturés à une teneur comprise entre 30 ppm en volume et 5 % volume, de préférence entre 0,05 et 3% volume. Généralement, les composés hydrocarbures insaturés comprennent essentiellement des produits hydrocarbures à courte chaine, typiquement C2, C3 ou C4, de la famille des alcènes, alcynes et polyinsaturés comme par exemple l'éthylène, l'acétylène, le butadiène.

### L'étape d'hydrogénation (étape a):

Dans le cadre de l'invention, l'hydrogénation peut être sélective, c'est-à-dire ne concerner que les alcynes et les polyinsaturés de type diènes mais pas les mono-oléfines. Toutefois, même si cela n'est généralement pas nécessaire, il n'y a aucun inconvénient à utiliser une hydrogénation totale, c'est-à-dire à hydrogéner tous les composés insaturés, y compris les mono-oléfines en paraffines.

Le catalyseur d'hydrogénation mis en oeuvre à l'étape a) comprend un métal choisi parmi le platine, le palladium, le nickel et le cobalt seuls ou en mélange et déposés sur un support poreux.

Selon une première variante du procédé selon l'invention, le catalyseur d'hydrogénation comprend du platine, et la teneur en platine, exprimé en métal, est typiquement comprise entre 0,02% poids et 4% poids par rapport au poids total de catalyseur. De préférence, la teneur en platine est comprise entre 0,05 et 3% poids, de manière plus préférée entre 0,1% et 2,5% poids par rapport au poids total de catalyseur

Selon une deuxième variante du procédé selon l'invention, le catalyseur d'hydrogénation comprend du palladium et la teneur en palladium, exprimé en métal, est comprise entre 0,05% poids et 5% poids par rapport au poids total de catalyseur. De préférence la teneur en palladium est comprise entre 0,05 et 3% poids, et de manière plus préférée entre 0,1% poids et 1% poids par rapport au poids total de catalyseur.

Selon une troisième variante du procédé selon l'invention, le catalyseur d'hydrogénation comprend du nickel et la teneur en nickel est généralement comprise entre 0,5% poids et 15% poids d'oxyde de nickel par rapport au poids total de catalyseur. De préférence, la teneur en oxyde de nickel est comprise entre 4% et 12% poids, de manière plus préférée entre 6% et 10% poids par rapport au poids total de catalyseur.

Selon une quatrième variante du procédé selon l'invention, le catalyseur d'hydrogénation comprend du cobalt et la teneur en cobalt est généralement comprise entre 0,5% poids et 15% poids d'oxyde de cobalt par rapport au poids total de catalyseur. De préférence, la teneur en oxyde de cobalt est comprise entre 1% et 10% poids, de manière plus préférée entre 2% et 4% poids par rapport au poids total de catalyseur.

Selon un mode de réalisation particulier, le catalyseur d'hydrogénation contient soit du platine, soit du palladium, soit du nickel, soit du cobalt et peut comprendre en outre du molybdène. Dans ce cas la teneur, exprimée en oxyde de molybdène dudit catalyseur est comprise entre 1% poids et 20% poids par rapport au poids total de catalyseur, de préférence comprise entre 6% et 18% poids, et de manière plus préférée entre 8% et 15% poids.

Le catalyseur de l'étape a) est un catalyseur qui comprend en outre un support poreux sur lequel sont déposés le ou les métaux ou précurseurs des métaux ou oxydes actifs en hydrogénation. Le support peut être choisi parmi les alumines, les silices, l'oxyde de titane, le carbure de silicium ou leurs mélanges.

Le support poreux est de préférence choisi parmi l'alumine, l'aluminate de nickel ou de cobalt, la silice, les silice-alumines, le carbure de silicium, l'oxyde de titane, ou leurs mélanges. On utilise, de manière préférée, de l'alumine pure ou de l'oxyde de titane.

Selon une variante très préférée, le support est constitué d'alumine gamma cubique ou d'alumine delta.

De façon plus préférée, le catalyseur d'hydrogénation mise en oeuvre à l'étape a) comprend du palladium. Selon un autre mode plus préféré, il comprend du nickel et du molybdène.

Le catalyseur selon l'invention peut être préparé au moyen de toute technique connue de l'homme du métier, et notamment par imprégnation des éléments métalliques sur le support sélectionné. Cette imprégnation peut par exemple être réalisée selon le mode connu de l'homme du métier sous la terminologie d'imprégnation à sec, dans lequel on introduit juste la quantité d'éléments désirés sous forme de sels solubles dans le solvant choisi, par exemple de l'eau déminéralisée, de façon à remplir aussi exactement que possible la porosité du support. Le support ainsi rempli par la solution est de préférence séché. Le support préféré est l'alumine ou l'oxyde de titane qui peuvent être préparés à partir de tout type de précurseurs et outils de mise en forme connus de l'homme de métier.

Après introduction des précurseurs des éléments métalliques, et éventuellement une mise en forme du catalyseur, celui-ci subi un traitement thermique qui comprend une étape de séchage suivie d'une calcination. Le séchage est généralement opéré sous air entre 20°C et 200°C, de préférence entre 40°C et 180°C. La calcination est généralement mise en oeuvre sous air ou sous oxygène dilué, et la température de traitement est généralement comprise entre 200°C et 550°C, de préférence entre 300°C et 500°C.

### L'étape d'hydrolyse (étape b) :

Le catalyseur d'hydrolyse mis en oeuvre à l'étape b) est un catalyseur qui comprend de l'alumine ou de l'oxyde de titane, de préférence de l'oxyde de titane. Le catalyseur selon l'invention peut inclure en outre au moins 1% poids, de préférence entre 0,5% poids et 10% poids, de manière plus préférée entre 1% poids et 5% poids d'au moins un sulfate ou silicate d'un élément alcalin ou alcalino-terreux ou d'une terre rare.

Ledit élément alcalin est préférentiellement choisi parmi le lithium, le sodium, le potassium et de manière plus préférée parmi le sodium ou le potassium. Ledit élément alcalino-terreux est de préférence choisi parmi le calcium, le baryum, le strontium et le magnésium. La terre rare est préférentiellement choisie parmi le lanthane, le cérium, le praséodyme ou le néodyme. De manière très préférée, ladite terre rare est le lanthane.

De manière plus préférée, ledit catalyseur comprend un silicate ou un sulfate de sodium, potassium, calcium ou baryum. De manière très préférée, il comprend du sulfate de calcium ou de baryum et de manière encore plus préférée du sulfate de calcium.

Selon un mode de réalisation particulier, le catalyseur d'hydrolyse comprend en outre un métal choisi parmi le nickel, le cobalt, le molybdène et le tungstène.

Selon une autre variante du procédé selon l'invention, le catalyseur d'hydrolyse mis en oeuvre à l'étape b) comprend 60% poids à 99,8% poids d'oxyde de titane ou d'alumine par rapport au poids du catalyseur et également un métal choisi parmi le nickel, le cobalt, le molybdène et le tungstène, seul ou en mélange.

Selon une variante préférée, le catalyseur d'hydrolyse comprend:
- de l'oxyde de titane ou de l'alumine une teneur comprise entre 60% et 99,8% poids par rapport au poids du catalyseur,
- au moins un métal choisi parmi le nickel, le cobalt, le molybdène et le tungstène à une teneur, exprimée en oxyde, comprise entre 0,2% et 40% poids, de préférence entre 1% à 25% par rapport au poids de catalyseur.

Selon une variante encore plus préférée, ledit catalyseur d'hydrolyse comprend du nickel et du molybdène ou du cobalt et du molybdène supportés sur un support en oxyde de titane.

Selon une autre variante très préférée, le catalyseur d'hydrolyse mise en oeuvre à l'étape b) comprend:
- du nickel à une teneur, exprimée % poids d'oxyde, comprise entre 0,5 et 15% poids, de préférence comprise entre 1% et 8% poids par rapport au poids du catalyseur;
- du cobalt à une teneur, exprimée % poids d'oxyde, comprise entre 0 et 10% poids, de préférence entre 0,5% et 5% poids par rapport au poids du catalyseur;
- du molybdène ou du tungstène à une teneur, exprimée % poids d'oxyde, comprise entre 1% et 18% poids, de préférence entre 5% et 15% poids par rapport au poids du catalyseur
- de l'oxyde de titane à une teneur, exprimée % en poids d'oxyde, comprise entre 60% et 98,5% par rapport au poids du catalyseur, de préférence comprise entre 70% et 98%.

De manière préférée, l'oxyde de titane est de structure cristallographique rutile ou anatase.

Le catalyseur peut être préparé au moyen de toute technique connue de l'homme du métier, et notamment par imprégnation de précurseurs de nickel, cobalt, molybdène, sulfate ou silicate d'un élément alcalin ou alcalino-terreux ou d'une terre rare sur le support qui a été préalablement mis en forme.

Cette imprégnation peut par exemple être réalisée selon le mode connu de l'homme du métier sous la terminologie "d'imprégnation à sec", dans lequel on introduit juste la quantité d'éléments désirés sous forme de sels solubles dans le solvant choisi, par exemple de l'eau déminéralisée, de façon à remplir aussi exactement que possible la porosité du support.

Le support imprégné peut ensuite être séché, de préférence à une température comprise entre 20°C et 200°C, de manière plus préférée comprise entre 40°C et 180°C. Une calcination est ensuite généralement mise en oeuvre sous air ou sous oxygène dilué, et la température de calcination est généralement comprise entre 200°C et 550°C, de préférence entre 300°C et 500°C.

### Description des figures

Ces aspects ainsi que d'autres aspects de l'invention seront clarifiés dans la description détaillée de modes de réalisation particuliers de l'invention, référence étant faite aux figures, dans lesquels:
- Fig.1 montre un premier mode de réalisation du procédé selon l'invention.
- Fig. 2 montre un second mode de réalisation du procédé selon l'invention.
- Fig. 3 montre un troisième mode de réalisation du procédé selon l'invention.

Les figures ne sont pas dessinées à l'échelle. Généralement, les éléments semblables sont dénotés par des références identiques dans les figures.

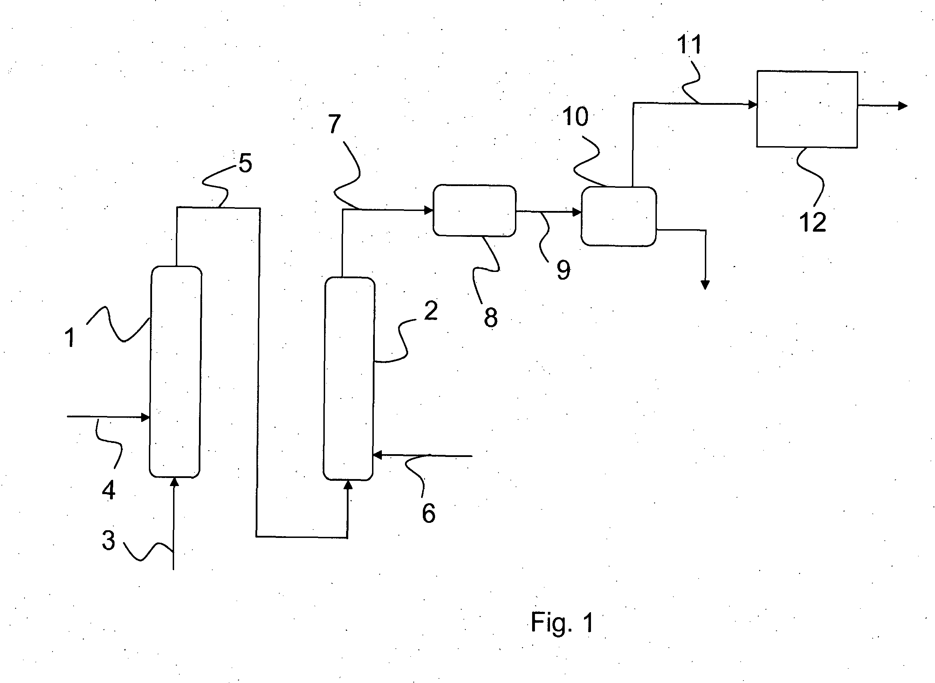

En référence à la figure 1, le procédé met en oeuvre un premier réacteur 1 dans lequel est présent un catalyseur d'hydrogénation des composés insaturés, de préférence un catalyseur d'hydrogénation sélective. Tout catalyseur d'hydrogénation décrit précédemment peut être utilisé dans ce mode de réalisation.

Comme montré dans la Fig. 1, la charge gazeuse à traiter est introduite dans le réacteur 1 via la ligne 3 tandis qu'une quantité d'hydrogène supplémentaire, en complément de l'hydrogène initialement présent dans le gaz à traiter, peut éventuellement être apportée dans le réacteur 1 grâce à la ligne 4.

La quantité d'hydrogène totale présente dans le gaz à traiter et éventuellement ajoutée est telle que le rapport molaire entre l'hydrogène et les composés hydrocarbures insaturés à hydrogéner soit supérieur à la stoechiométrie et de préférence compris entre 1 et 3000 moles par moles et de préférence compris entre 300 et 2000 moles par moles.

L'étape d'hydrogénation est conduite généralement à une pression comprise entre 0,1 et 5 MPa, de préférence comprise entre 0,5 et 3 MPa, à une température comprise entre 100 et 400°C, de préférence entre 150°C et 250°C, et un volume de catalyseur en rapport avec la quantité de gaz à traiter avec 1 m³ de catalyseur pour 1000 à 4000 Nm³/h de gaz à traiter, soit une VVH comprise entre 1000 et 4000 h⁻¹.

En référence à la figure 1, l'effluent issu du réacteur d'hydrogénation est ensuite envoyé dans le réacteur d'hydrolyse 2, via la ligne 5, dans lequel est réalisée la conversion des composés sulfurés COS et CS₂ en H₂S sur un catalyseur spécifique en présence d'eau.

Si la teneur en eau de la charge n'est pas suffisante, de l'eau additionnelle peut être introduite via la ligne 6, afin de réaliser l'hydrolyse avec un excès d'eau par rapport aux molécules hydrolysables (COS, CS₂, HCN).

Tout catalyseur d'hydrolyse décrit précédemment peut être utilisé dans ce mode de réalisation.

Les réactions mises en jeu lors de cette étape peuvent être représentées par les réactions suivantes:

COS ± H₂O → CO₂ + H₂S

CS₂ + 2 H₂O → 2 H₂S + CO₂

L'étape d'hydrolyse est typiquement réalisée à une pression comprise entre 0,1 et 5 MPa, de préférence comprise entre 0,5 et 3 MPa, à une température comprise entre 100 et 400°C, de préférence comprise entre 150°C et 250°C, et un volume de catalyseur en rapport avec la quantité de gaz à traiter de 1 m³ de catalyseur pour 1000 à 4000 Nm³/h de gaz à traiter, soit une VVH comprise entre 1000 et 4000 h⁻¹.

On opère en présence d'un excès d'eau par rapport aux molécules à hydrolyser. De préférence, on opère avec un rapport molaire eau/produits hydrolysables compris entre 5 et 1000 moles pour moles et de manière plus préféré compris entre 10 et 500 moles pour moles.

L'effluent gazeux traité dans le réacteur d'hydrolyse 2 est alors extrait et conduit par la ligne 7 vers un échangeur de chaleur 8, par exemple un aéroréfrigérant, de manière à refroidir le gaz traité. Le gaz traité et refroidi est transféré par la ligne 9 dans un séparateur liquide/gaz 10. L'eau liquide de condensation est récupérée en fond du séparateur 10, tandis que le gaz appauvri en H₂O et contenant l'H₂S est amené grâce à la ligne 11 vers une unité de traitement 12 qui peut être par exemple une unité de piégeage de l'H₂S ou une unité de conversion de l'H₂S qui réalise par exemple l'oxydation de l'H₂S pour former du soufre élémentaire :

2 H₂S + SO₂ → 3 S + 2 H₂O

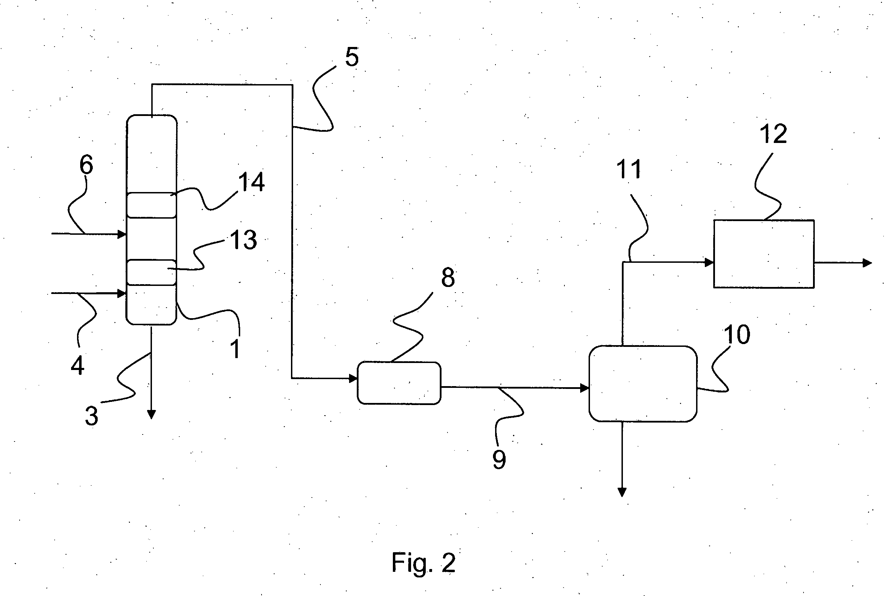

Selon l'invention, les deux réactions d'hydrogénation et d'hydrolyse de la charge à traiter peuvent être réalisées dans un même réacteur comprenant un premier lit de catalyseurs d'hydrogénation et un second lit de catalyseur d'hydrolyse, les lits étant agencés l'un par rapport à l'autre dans le réacteur de sorte que la charge gazeuse à traiter rencontre le lit de catalyseur d'hydrogénation avant le lit de catalyseur d'hydrolyse, comme représenté sur la figure 2.

En référence à la figure 2, le second mode de réalisation met en jeu un unique réacteur 1 dans lequel sont réalisées les réactions d'hydrogénation et d'hydrolyse catalytique. A cet effet, le réacteur comprend deux lits de catalyseurs 13 et 14 respectivement d'hydrogénation et d'hydrolyse. Ces deux lits peuvent être séparés l'un de l'autre par un espace interne ou au contraire être consécutifs sans aucun espace intermédiaire. Les lits de catalyseur 13 et 14 sont agencés dans le réacteur 1 de manière à ce que la charge à traiter rencontre d'abord le lit catalytique d'hydrogénation 13 et ensuite le lit catalytique d'hydrolyse.

Afin de réaliser l'hydrogénation de la charge gazeuse qui est introduite via la ligne 3, un apport d'hydrogène supplémentaire peut éventuellement être réalisé au moyen de la ligne 4 situé en amont du lit de catalyseur 13. Lorsque cela est nécessaire, un espace interne sépare les lits catalytiques 13 et 14, afin de positionner dans cet espace d'un point d'injection d'un supplément d'eau nécessaire à la réaction d'hydrolyse via la ligne 6.

Les conditions opératoires utilisées pour les deux réactions catalytiques et décrites en référence à la figure 1 sont applicables dans ce second mode de réalisation. Tout catalyseur d'hydrogénation ou d'hydrolyse décrit précédemment peut être également utilisé dans ce mode de réalisation.

De façon similaire au mode de réalisation de la figure 1, l'effluent gazeux après hydrolyse catalytique est évacué du réacteur 1 et envoyé par la ligne 5 dans un échangeur de chaleur 8 puis dans un ballon séparateur 10 via la ligne 9. Du ballon séparateur 10 est extrait en fond l'eau de condensation et en tête un gaz chargé en H₂S qui est transféré vers une unité de piégeage ou de conversion 12 de l'H₂S.

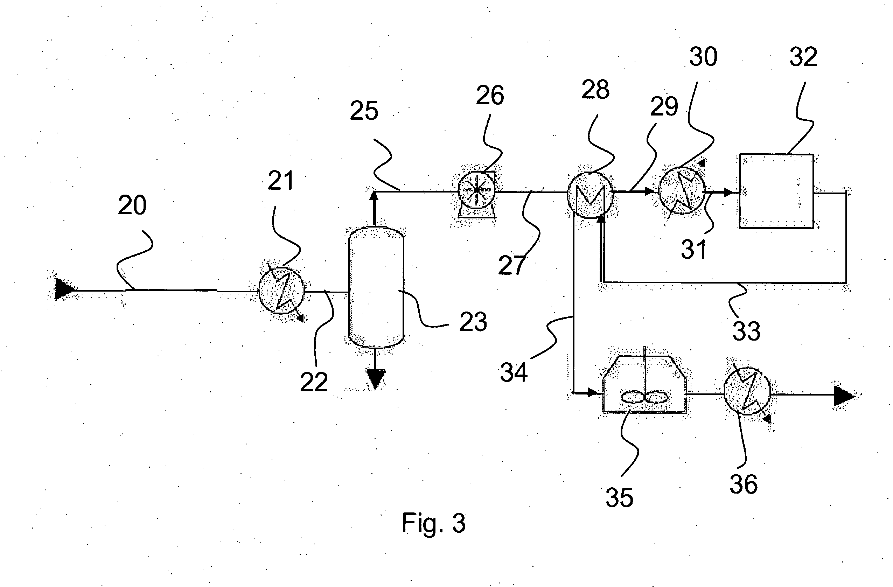

Le troisième mode de réalisation du procédé selon l'invention est représenté à la figure 3 et diffère essentiellement des modes de réalisation des figures 1 et 2 en ce qu'il comporte un étape préalable de séparation gaz/liquide réalisée sur le gaz à traiter. Il est en effet intéressant d'enlever essentiellement de l'eau excédentaire et/ou éventuellement des composés organiques liquides dissous ou non dans la phase gazeuse afin de diminuer le volume de charge à traiter, ceci tout en conservant un excès d'eau pour réaliser la réaction d'hydrolyse.

Dans ce cas, comme montré sur la figure 3, le gaz à traiter qui est généralement chaud est envoyé vers un échangeur à chaleur 21 par la ligne 20 où il est refroidi, puis envoyé via la ligne 22 vers un ballon séparateur 23. Dans le ballon 23, on sépare deux phases à savoir une phase gazeuse en tête et une phase liquide en fond qui contient l'eau de la charge. Le gaz séparé de ce liquide et éventuellement d'une partie de l'eau dissoute issu du ballon séparateur 23 est envoyé par le conduit 25 dans un compresseur 26 pour y être comprimé.

Le gaz comprimé subit éventuellement, comme montré sur la figure 3, une étape de chauffage à travers l'échangeur de chaleur optionnel 28 qui, lorsqu'il est présent est alimenté par un fluide chaud. Ce fluide chaud est, de préférence et selon l'exemple de la figure 3, l'effluent gazeux chaud issu de la zone réactionnelle 32 d'hydrogénation et d'hydrolyse. Le gaz comprimé préalablement chauffé peut éventuellement être porté à la température opérationnelle au moyen d'une unité de chauffe optionnelle 30, par exemple un échangeur, avant d'être introduit dans la zone réactionnelle 32 où sont réalisées les réactions d'hydrogénation et d'hydrolyse selon le procédé de l'invention. L'effluent chaud issu de la zone réactionnelle 32 est évacué par la ligne 33 et introduit dans l'échangeur de chaleur 28 pour réchauffer le gaz à traiter. Après avoir été refroidi au contact du gaz à traiter, le gaz traité riche en H₂S peut être éventuellement envoyé par la ligne 34 dans un second condenseur 35 optionnel, par exemple un aéroréfrigérant et/ou éventuellement dans un échangeur de chaleur 36 également optionnel. Ce refroidissement permet de régler la température du flux à une température compatible avec d'éventuels traitements en aval. Ainsi, le flux refroidi peut être notamment traité ensuite dans une unité (non représentée) de piégeage de l'H₂S ou de conversion de l'H₂S en soufre élémentaire.

### Exemples

Les exemples 1 (comparatif), 2 et 3 (selon l'invention) se rapportent à l'efficacité de la conversion des composés soufrés sous forme H₂S d'une charge A typique dont la composition est donnée dans le Tableau 1. La charge A correspond à un effluent issu d'une unité de production de noir de charbon et comprend des composés soufrés et azotés (COS, CS₂ et HCN) et de l'acétylène. L'exemple 1 est réalisé selon le procédé décrit dans le brevet US 5,466,427.

Les exemples 2 et 3 sont réalisés selon le procédé de l'invention comprenant au moins une première étape a) d'hydrogénation des composés hydrocarbures insaturés présents dans la charge A suivie d'une étape b) d'hydrolyse catalytique des composés soufrés et azotés (COS et/ou CS₂ et HCN) présents dans l'effluent issu de l'étape a).

**Tableau 1. Composition de la charge A**

| Composition de la charge A | Teneur (% volume) |
|---|---|
| CS₂ | 0,08 |
| HCN | 0,05 |
| COS | 0,013 |
| SO₂ | 0,007 |
| H₂S | 0,15 |
| CH₄ | 0,3 |
| C₂H₂ | 0,3 |
| CO | 8 |
| H₂ | 9 |
| CO₂ | 2 |
| H₂O | 45 |
| O₂ | 0,1 |
| N₂ | 35 |

La charge gazeuse à traiter contient donc une quantité non négligeable d'acétylène à hauteur de 0,3% en volume.

### Exemple 1 (comparatif)

La charge A est directement envoyée avec de l'eau dans un réacteur d'hydrolyse des composés COS et CS₂ (dimension du réacteur 2 cm de diamètre, 10 cm de haut) renfermant un catalyseur préparé par imprégnation d'un support à base d'oxyde de titane TiO₂ sous forme d'extrudé cylindrique de 3,2 mm de diamètre et de 6,7 mm de longueur moyenne, sur lequel est déposé du nickel et du molybdène. Le catalyseur a la composition suivante: 2,5% poids d'oxyde de nickel (NiO), 9,0% poids de trioxyde de molybdène (MoO₃) et 88,5% poids d'oxyde de titane. Les % poids sont exprimés par rapport au poids total du catalyseur.

Les conditions opératoires pour la réaction d'hydrolyse catalytique sont les suivantes:
- température (°C): 220
- pression (MPa) : 0,2
- VVH (h⁻¹): 3600
- teneur totale en eau : 45% volume

La composition de l'effluent à la sortie du réacteur est analysée (par chromatographie en phase gazeuse). Les résultats obtenus après 48h d'opération sont présentés dans le Tableau 2.

**Tableau 2: Composition de l'effluent gazeux issu de l'étape d'hydrolyse**

| Composition de l'effluent à la sortie du réacteur | Teneur (% volume) |
|---|---|
| CS₂ | 0,07 |
| HCN | 0,03 |
| COS | 0,005 |
| SO₂ | 0,005 |
| H₂S | 0,16 |
| CH₄ | 0,3 |
| C₂H₂ | 0,3 |
| CO | 8 |
| H₂ | 9 |
| CO₂ | 2 |
| H₂O | 45 |
| O₂ | non déterminé |
| N₂ | 35 |
| NH₃ | 0,02 |

Après 48h de fonctionnement, on observe un abaissement de la teneur en CS₂ de l'ordre d'à peine 12%. En revanche on constate une diminution de la teneur en COS de 61%. Ainsi le catalyseur d'hydrolyse manifeste une faible activité d'hydrolyse du disulfure de carbone lorsque la réaction est réalisée avec un gaz comprenant des composés organiques insaturés (dans le cas présent de l'acétylène).

### Exemple 2 (selon l'invention)

La même charge A dont la composition a été donnée dans le Tableau 1 est d'abord envoyée dans un premier réacteur d'hydrogénation selon l'étape a) de l'invention. Le catalyseur d'hydrogénation utilisé lors de l'étape a) est constitué de 0,28% poids de Pd sur un support constitué d'alumine gamma agglomérée sous forme de billes de 1,7mm de diamètre. L'étape a) est mise en oeuvre dans les conditions opératoires suivantes:
- température (°C): 220
- pression (MPa) : 0,2
- VVH (h⁻¹): 3200
- teneur en H₂ dans la charge : 9% volume, on n'ajoute donc pas d'hydrogène supplémentaire.

L'effluent issu de l'étape a) est analysée après 48h d'opération selon la méthode décrite dans l'exemple 1 et présente une composition donnée dans le Tableau 3.

**Tableau 3 : composition de l'effluent de l'étape d'hydrogénation**

| Effluent issu de l'étape a) | Teneur (% volume) |
|---|---|
| CS₂ | 0,07 |
| HCN | 0,005 |
| COS | 0,008 |
| SO₂ | 0,001 |
| H₂S | 0,18 |
| CH₄ | 0,3 |
| C₂H₂ | < 0,01 |
| C₂H₆ | 0,3 |
| CO | 7,8 |
| H₂ | 9 |
| CO₂ | 2,1 |
| H₂O | 45 |
| O₂ | < 0,001 |
| N₂ | 35 |

Ainsi l'étape d'hydrogénation a permis de réduire notablement la concentration en acétylène et former de l'éthane correspondant.

L'effluent issu de l'étape a) est ensuite dirigé vers un deuxième réacteur selon l'étape b) de l'invention. Le catalyseur utilisé lors de l'étape b) est constitué de 91% poids de TiO₂ et 9% poids de CaSO₄. L'étape b) est mise en oeuvre dans les conditions opératoires suivantes:
- température (°C): 220
- pression (MPa) : 0,2
- VVH (h⁻¹): 2000
- teneur en eau dans la charge : 45% volume (on n'ajoute donc pas d'eau supplémentaire)

La composition de l'effluent issu de l'étape b) est donnée dans le tableau 4.

**Tableau 4 : Composition de l'effluent de l'étape d'hydrolyse**

| Effluent issu de l'étape b) | Teneur (% volume) |
|---|---|
| CS₂ | 0,01 |
| HCN | < 0,001 |
| COS | 0,005 |
| SO₂ | 0,001 |
| H₂S | 0,30 |
| CH₄ | 0,3 |
| C₂H₂ | < 0,01 |
| C₂H₆ | 0,3 |
| CO | 7,8 |
| H₂ | 9 |
| CO₂ | 2,1 |
| H₂O | 45 |
| O₂ | < 0,001 |
| N₂ | 35 |

On observe que l'étape d'hydrogénation effectuée sur le gaz résiduaire a un effet positif sur le rendement d'hydrolyse des composés CS₂. Ainsi on obtient un abaissement de la teneur en CS₂ de l'ordre de 86%. On peut donc conclure qu'un pré-traitement du gaz en vue de réduire la concentration en composés organiques insaturés permet de maintenir une meilleure activité d'hydrolyse des disulfures de carbone.

### Exemple 3 (selon l'invention)

La charge A décrite dans le Tableau 1 est d'abord envoyée dans un premier réacteur selon l'étape a) de l'invention. Le catalyseur d'hydrogénation utilisé lors de l'étape a) est constitué de 0,28 % poids de Pd sur un support constitué d'alumine gamma agglomérée sous forme de billes de 1,7 mm de diamètre. L'étape a) est mise en oeuvre dans les conditions opératoires suivantes:
- température (°C): 220
- pression (MPa) : 0,2
- VVH (h⁻¹): 3200
- teneur en H₂ dans la charge : 9% volume, on n'ajoute donc pas d'hydrogène supplémentaire.

La composition de l'effluent issu de l'étape a) est analysée après 48h d'opération selon la méthode décrite dans l'exemple 1. La composition de l'effluent issu de l'étape a) est donnée dans le Tableau 5.

**Tableau 5 : Composition de l'effluent de l'étape d'hydrogénation**

| Effluent issu de l'étape a) | Teneur (% volume) |
|---|---|
| CS₂ | 0,07 |
| HCN | 0,005 |
| COS | 0,008 |
| SO₂ | 0,001 |
| H₂S | 0,18 |
| CH₄ | 0,3 |
| C₂H₂ | < 0,01 |
| C₂H₆ | 0,3 |
| CO | 7,8 |
| H₂ | 9 |
| CO₂ | 2,1 |
| H₂O | 45 |
| O₂ | < 0,001 |
| N₂ | 35 |

Le traitement préalable du gaz de la charge par hydrogénation permet ainsi de transformer l'acétylène en éthane et par conséquent d'amener la teneur en acétylène à une valeur inférieure à 0,01% en volume.

L'effluent issu de l'étape a) est ensuite dirigé traité dans un deuxième réacteur selon l'étape b) de l'invention. Le catalyseur utilisé lors de l'étape b) est celui présenté dans l'exemple 1. Le catalyseur a la composition suivante (exprimés par rapport au poids total du catalyseur): 2,5% poids d'oxyde de nickel (NiO), 9,0% poids de trioxyde de molybdène (MoO₃) et 88,5% poids d'oxyde de titane.

Les conditions opératoires de cette étape b) sont les suivantes :
- température (°C): 220
- pression (MPa) : 0,2
- VVH (h⁻¹): 2000
- teneur en eau dans la charge : 45% volume on n'ajoute donc pas d'eau supplémentaire

La composition de l'effluent issu de l'étape b) est analysée selon la méthode décrite à l'exemple 1. Les résultats sont présentés dans le tableau 6.

**Tableau 6 : Composition de l'effluent de l'étape d'hydrolyse**

| Effluent issu de l'étape b) | Teneur (% volume) |
|---|---|
| CS₂ | 0,01 |
| HCN | < 0,001 |
| COS | 0,005 |
| SO₂ | 0,001 |
| H₂S | 0,30 |
| CH₄ | 0,3 |
| C₂H₂ | < 0,01 |
| C₂H₆ | 0,3 |
| CO | 4,6 |
| H₂ | 12,2 |
| CO₂ | 5,3 |
| H₂O | 42 |
| O₂ | < 0,001 |
| N₂ | 35 |

Les analyses indique que l'étape préalable d'hydrogénation en vue de saturer les composés organiques insaturés permet de préserver les performances catalytiques du catalyseur en particulier l'activité en d'hydrolyse vis-à-vis des disulfures de carbone.

En effet là encore, on obtient un abaissement de la teneur en C2S de l'ordre de 87% et en COS de l'ordre de 61%.

## Revendications

1. Procédé de traitement d'un gaz comprenant de 10 ppm en volume à 0,5% volume d'au moins l'un des composés COS et CS₂ et de 30 ppm en volume à 5% volume de composés hydrocarbures insaturés, ledit procédé comprenant les étapes suivantes:
a) on effectue une hydrogénation (1) des composés hydrocarbures insaturés en paraffines, en mettant en contact ledit gaz avec un catalyseur d'hydrogénation en présence d'hydrogène à une température comprise entre 100 et 400°C, de manière à fournir un effluent appauvri en composés hydrocarbures insaturés, le catalyseur d'hydrogénation comprenant au moins un métal choisi parmi le palladium, le platine, le nickel et le cobalt déposé sur un support poreux,
b) on effectue une hydrolyse catalytique (2) en présence d'eau du COS et/ou du CS₂ présents dans l'effluent issu de l'étape a) de manière à fournir un effluent riche en H₂S, en mettant en contact l'effluent issu de l'étape a) avec un catalyseur d'hydrolyse, le catalyseur d'hydrolyse comprenant de l'alumine ou de l'oxyde de titane.

2. Procédé selon la revendication 1, dans lequel le catalyseur d'hydrogénation comprend une teneur en platine, exprimée en métal, comprise entre 0,02% poids et 4% poids par rapport au poids de catalyseur.

3. Procédé selon la revendication 1, dans lequel le catalyseur d'hydrogénation comprend une teneur en palladium, exprimée en métal, comprise entre 0,05% poids et 5% poids par rapport au poids de catalyseur.

4. Procédé selon la revendication 1, dans lequel le catalyseur d'hydrogénation comprend une teneur en nickel, exprimé en oxyde, comprise entre 0,5% poids et 15% poids par rapport au poids de catalyseur.

5. Procédé selon la revendication 1, dans lequel le catalyseur d'hydrogénation comprend une teneur en cobalt, exprimée en oxyde, comprise entre 0,5% poids et 15% poids par rapport au poids de catalyseur.

6. Procédé selon l'une des revendications précédentes, dans lequel le catalyseur d'hydrogénation comprend en outre du molybdène à une teneur exprimée en oxyde, comprise entre 1% et 20% poids par rapport au poids de catalyseur.

7. Procédé selon l'une des revendications précédentes, dans lequel le catalyseur d'hydrolyse comprend en outre au moins 1% poids, de préférence entre 0,5% poids et 10% poids d'au moins un sulfate ou silicate d'un élément alcalin ou alcalino-terreux ou d'une terre rare.

8. Procédé selon l'une des revendications 1 à 6, dans lequel le catalyseur d'hydrolyse comprend en outre un métal choisi parmi le nickel, le cobalt, le molybdène et le tungstène.

9. Procédé selon la revendication 8, dans lequel le catalyseur comprend:
• de l'oxyde de titane ou d'alumine à une teneur comprise entre 60% et 99,8% poids par rapport au poids du catalyseur,
• au moins un métal choisi parmi le nickel, le cobalt, le molybdène et le tungstène à une teneur, exprimée en oxyde, comprise entre 0,2% et 40% poids, de préférence entre 1% à 25% par rapport au poids de catalyseur.

10. Procédé selon l'une quelconque des revendications précédentes, dans lequel les étapes a) et b) sont réalisées dans un même réacteur comprenant un lit de catalyseurs d'hydrogénation (13) et un lit de catalyseurs d'hydrolyse (14), les lits (13, 14) étant agencés l'un par rapport à l'autre dans le réacteur de sorte que le gaz à traiter rencontre le lit de catalyseurs d'hydrogénation (13) avant le lit de catalyseurs d'hydrolyse (14).

11. Procédé selon l'une quelconque des revendications précédentes dans lequel l'étape a) est effectuée à une pression comprise entre 0,1 et 5 MPa et un VVH compris entre 1000 et 4000 h⁻¹.

12. Procédé selon l'une quelconque des revendications précédentes, dans lequel l'étape b) est effectuée à une pression comprise entre 0,1 et 5 MPa et un VVH compris entre 1000 et 4000 h⁻¹.

13. Procédé selon l'une quelconque des revendications précédentes comprenant une étape c) dans laquelle on effectue un traitement de l'effluent riche en H₂S issu de l'étape b) dans une unité de piégeage de l'H₂S ou de conversion de l'H₂S en soufre élémentaire.

14. Procédé selon l'une quelconque des revendications précédentes dans lequel on effectue une séparation liquide/gaz du gaz à traiter avant d'effectuer l'étape a) d'hydrogénation.

15. Procédé selon l'une quelconque des revendications précédentes dans lequel le gaz à traiter est issus d'unités de gazéification du charbon ou de petcoke ou encore de biomasse ou des fours de calcination des unités de fabrication de noir de carbone.

## Patentansprüche

1. Verfahren zur Behandlung eines Gases, umfassend 10 Vol.-ppm bis 0,5 Vol.-% mindestens einer der Verbindungen COS und CS₂ und 30 Vol.-ppm bis 5 Vol.-% ungesättigte Kohlenwasserstoffverbindungen, wobei das Verfahren die folgenden Schritte umfasst:
a) Hydrieren (1) der ungesättigten paraffinischen Kohlenwasserstoffverbindungen, indem das Gas mit einem Hydrierkatalysator in Gegenwart von Wasserstoff bei einer Temperatur im Bereich zwischen 100 und 400 °C in Kontakt gebracht wird, um einen Abfluss bereitzustellen, der an ungesättigten Kohlenwasserstoffverbindungen verarmt ist, wobei der Hydrierkatalysator mindestens ein Metall, ausgewählt aus Palladium, Platin, Nickel und Cobalt, aufgebracht auf einem porösen Träger, umfasst,
b) Durchführen einer katalytischen Hydrolyse (2) von COS und/oder CS₂ in Gegenwart von Wasser, die in dem aus Schritt a) stammenden Abfluss vorhanden sind, um einen Abfluss bereitzustellen, der reich an H₂S ist, indem der aus Schritt a) stammende Abfluss mit einem Hydrolysekatalysator in Kontakt gebracht wird, wobei der Hydrolysekatalysator Aluminiumoxid oder Titanoxid umfasst.

2. Verfahren nach Anspruch 1, wobei der Hydrierkatalysator einen Platingehalt, als Metall gerechnet, im Bereich zwischen 0,02 Gew.-% und 4 Gew.-%, bezogen auf das Gewicht des Katalysators, umfasst.

3. Verfahren nach Anspruch 1, wobei der Hydrierkatalysator einen Palladiumgehalt, als Metall gerechnet, im Bereich zwischen 0,05 Gew.-% und 5 Gew.-%, bezogen auf das Gewicht des Katalysators, umfasst.

4. Verfahren nach Anspruch 1, wobei der Hydrierkatalysator einen Nickelgehalt, als Oxid gerechnet, im Bereich zwischen 0,5 Gew.-% und 15 Gew.-%, bezogen auf das Gewicht des Katalysators, umfasst.

5. Verfahren nach Anspruch 1, wobei der Hydrierkatalysator einen Cobaltgehalt, als Oxid gerechnet, im Bereich zwischen 0,5 Gew.-% und 15 Gew.-%, bezogen auf das Gewicht des Katalysators, umfasst.

6. Verfahren nach einem der vorhergehenden Ansprüche, wobei der Hydrierkatalysator ferner einen Molybdängehalt, als Oxid gerechnet, im Bereich zwischen 1 Gew.-% und 20 Gew.-%, bezogen auf das Gewicht des Katalysators, umfasst.

7. Verfahren nach einem der vorhergehenden Ansprüche, wobei der Hydrolysekatalysator ferner mindestens 1 Gew.-%, bevorzugt zwischen 0,5 Gew.-% und 10 Gew.-% mindestens eines Sulfats oder Silikats eines Alkali- oder Erdalkali- oder Seltenerdenelements umfasst.

8. Verfahren nach einem der Ansprüche 1 bis 6, wobei der Hydrolysekatalysator ferner ein Metall, ausgewählt aus Nickel, Cobalt, Molybdän und Wolfram, umfasst.

9. Verfahren nach Anspruch 8, wobei der Katalysator umfasst:
• Titanoxid oder Aluminiumoxid in einem Gehalt im Bereich zwischen 60 Gew.-% und 99,8 Gew.-%, bezogen auf das Gewicht des Katalysators,
• mindestens ein Metall, ausgewählt aus Nickel, Cobalt, Molybdän und Wolfram, in einem Gehalt, als Oxid gerechnet, im Bereich zwischen 0,2 Gew.-% und 40 Gew.-%, bevorzugt zwischen 1 % und 25 %, bezogen auf das Gewicht des Katalysators.

10. Verfahren nach einem der vorhergehenden Ansprüche, wobei die Schritte a) und b) in dem gleichen Reaktor ausgeführt werden, wobei der Reaktor ein Hydrierkatalysatorenbett (13) und ein Hydrolysekatalysatorenbett (14) umfasst, wobei die Betten (13, 14) in dem Reaktor derart zueinander angeordnet sind, dass das aufzubereitende Gas auf das Hydrierkatalysatorenbett (13) trifft bevor es auf das Hydrolysekatalysatorenbett (14) trifft.

11. Verfahren nach einem der vorhergehenden Ansprüche, wobei Schritt a) bei einem Druck im Bereich zwischen 0,1 und 5 MPa und einer HSV im Bereich zwischen 1.000 und 4.000 h⁻¹ durchgeführt wird.

12. Verfahren nach einem der vorhergehenden Ansprüche, wobei Schritt b) bei einem Druck im Bereich zwischen 0,1 und 5 MPa und einer HSV im Bereich zwischen 1.000 und 4.000 h⁻¹ durchgeführt wird.

13. Verfahren nach einem der vorhergehenden Ansprüche, umfassend einen Schritt c), wobei eine Aufarbeitung des aus Schritt b) kommenden Abflusses, der reich an H₂S ist, in einer Einheit zum Abtrennen von H₂S oder zur Umwandlung von H₂S in elementaren Schwefel durchgeführt wird.

14. Verfahren nach einem der vorhergehenden Ansprüche, wobei eine Gas-/Flüssigkeitsabscheidung des aufzuarbeitenden Gases durchgeführt wird, bevor Schritt a) zur Hydrierung durchgeführt wird.

15. Verfahren nach einem der vorhergehenden Ansprüche, wobei das aufzuarbeitende Gas aus Einheiten zur Vergasung von Kohle oder Petrolkoks oder auch von Biomasse oder aus Brennöfen von Einheiten zur Herstellung von Industrieruß stammt.

## Claims

1. Process for treating a gas containing from 10 ppm by volume to 0.5 volume % of at least one of the compounds COS and CS₂ and from 30 ppm by volume to 5 volume % of unsaturated hydrocarbon compounds, the said process comprising the following stages:
a) a hydrogenation (1) of the unsaturated hydrocarbon compounds to paraffins is carried out by contacting the said gas with a hydrogenation catalyst in the presence of hydrogen at a temperature between 100 and 400°C, so as to provide a gaseous effluent depleted in unsaturated hydrocarbon compounds, the hydrogenation catalyst comprising at least one metal selected from palladium, platinum, nickel and cobalt deposited on a porous support,
b) a catalytic hydrolysis (2) of COS and/or CS₂ present in the gaseous effluent from stage a) is carried out in the presence of water so as to provide a gaseous effluent rich in H₂S by contacting the said gaseous effluent from stage a) with a hydrolysis catalyst, the hydrolysis catalyst comprising alumina or titanium oxide.

2. Process according to claim 1, in which the hydrogenation catalyst contains platinum, expressed as metal, in an amount between 0.02 wt.% and 4 wt.% with respect to the weight of the catalyst.

3. Process according to claim 1, in which the hydrogenation catalyst contains palladium, expressed as metal, in an amount between 0.05 wt.% and 5 wt.% with respect to the weight of the catalyst.

4. Process according to claim 1, in which the hydrogenation catalyst contains nickel in an amount, expressed as oxide, between 0.5 wt.% and 15 wt.% with respect to the weight of the catalyst.

5. Process according to claim 1, in which the hydrogenation catalyst contains cobalt in an amount, expressed as oxide, between 0.5 wt.% and 15 wt.% with respect to the weight of the catalyst.

6. Process according to any one of the preceding claims, in which the hydrogenation catalyst also contains molybdenum in an amount, expressed as oxide, between 1 wt.% and 20 wt.% with respect to the weight of the catalyst.

7. Process according to any one of the preceding claims, in which the hydrolysis catalyst also contains at least 1 wt.%, preferably between 0.5 wt.% and 10 wt.%, of at least one sulphate or silicate of an alkali metal or alkaline earth metal or of a rare earth.

8. Process according to any one of claims 1 to 6, in which the hydrolysis catalyst also contains a metal chosen from nickel, cobalt, molybdenum and tungsten.

9. Process according to claim 8, in which the catalyst comprises:
• titanium oxide or alumina in an amount between 60 wt.% and 99.8 wt.% with respect to the weight of the catalyst,
• at least one metal chosen from nickel, cobalt, molybdenum and tungsten in an amount, expressed as oxide, between 0.2 wt.% and 40 wt.%, preferably between 1 wt.% to 25 wt.% with respect to the weight of the catalyst.

10. Process according to any one of the preceding claims, in which the stages a) and b) are carried out in the same reactor comprising a hydrogenation catalyst bed (13) and a hydrolysis catalyst bed (14), the beds (13, 14) being arranged with respect to one another in the reactor so that the gas to be treated comes in to contact with the hydrogenation catalyst bed (13) before the hydrolysis catalyst bed (14).

11. Process according to any one of the preceding claims, in which stage a) is carried out at a pressure between 0.1 and 5 MPa and a HSV between 1000 and 4000 h⁻¹.

12. Process according to any one of the preceding claims, in which stage b) is carried out at a pressure between 0.1 and 5 MPa and a HSV between 1000 and 4000 h⁻¹.

13. Process according to any one of the preceding claims, comprising a stage c) in which the effluent rich in H₂S leaving the stage b) is treated in a unit for trapping H₂S or for converting H₂S into elementary sulphur.

14. Process according to any one of the preceding claims, in which a liquid/gas separation of the gas to be treated is implemented before carrying out the hydrogenation stage a).

15. Process according to any one of the preceding claims, in which the gas to be treated is obtained from coal or petcoke or even biomass gasification units or from calcination furnaces of carbon black production units.
